Europäisches Patentamt

European Patent Office

Office européen des brevets

(19)

(11) Publication number: **0 086 338**
**A1**

## EUROPEAN PATENT APPLICATION

(21) Application number: 83100329.8

(22) Date of filing: 15.01.83

(51) Int. Cl.³: **A 61 M 25/00,** A 61 B 1/00

(30) Priority: 20.01.82 US 340952

(43) Date of publication of application: 24.08.83
Bulletin 83/34

(84) Designated Contracting States: **AT BE CH DE FR GB IT LI LU NL SE**

(71) Applicant: **SORENSON RESEARCH CO. INC.,**
4455 Atherton, Salt Lake City Utah 84107 (US)

(72) Inventor: **Wonder, Terry M., 4220 South 500 East, Salt Lake City Utah 84107 (US)**
Inventor: **Dixon, John A., 875 Donner Way, Salt Lake City Utah 84108 (US)**

(74) Representative: **Modiano, Guido et al, MODIANO, JOSIF, PISANTY & STAUB Modiano & Associati Via Meravigli, 16, I-20123 Milan (IT)**

(54) Method and apparatus for translating and positioning a catheter.

(57) A catheter assembly (10), includes a flexible inner catheter tube (11) which is telescopically translatable through a shorter, outer sheath tube (12). The inner catheter tube (11) has an injection needle (19) projection from its distal end (13) which is retracted into the open distal end (14) of the sheath tube (12) as the sheath tube traverses the endoscope or other tubular passage. Upon reaching the desired location, the needle (19) and the distal end (13) of the inner catheter tube (11) are projected from the distal end (14) of the sheath (12) by applying an axial force to the catheter tube at a location beyond the proximal end (17) of the sheath (12). A positional locking mechanism includes an elastomeric washer (30) which concentrically slidably engages the catheter tube (11) at a location beyond the proximal end (17) of the sheath (12). The washer (30) is selectively axially compressed between two locking members (25, 21) which are disposed about the sheath (12) and catheter tube (11), respectively. The washer (30) is radially constricted so that the axial compression produces a radial compression which results in a gripping action on the catheter tube (11).

The present invention relates to catheters in general and, more particularly to a method and apparatus for facilitating translation and positionally locking a catheter. In a broader sense the present invention also relates to an improved locking mechanism which permits a flexible tube, which is telescopically and slidably mounted in a sheath tube, to be locked in position to prevent axial movement of the flexible tube with respect to the sheath tube.

In the course of certain medical procedures involving an endoscopic device (i.e. gastroscope, proctoscope, laparoscope, arthoscope, etc.), it is sometimes desirable to inject a medicament directly into organ tissue being visualized without resorting to any additional invasive or surgical procedure. In order to accomplish this, various prior art injection catheter devices have been developed which generally consist of a single lumen catheter with a needle attached at one end and a syringe attachment at the other. Such a device is intended to be inserted through one of the operative channels provided in an endoscope, thus providing an injection needle at the endoscope's distal end which can be controlled extracorporeally by the physician. On the surface this would seem to provide a satisfactory solution to the problem; in practice, however, the difficulty in threading these devices through the endoscope all but precludes their use. If the injection catheter is inserted in a forward manner (i.e., needle first, starting from the proximal or physician end of the endoscope) the sharpened needle point repeatedly snags on the soft elastomeric lining of the operative channel, thereby damaging the lining, dulling and contaminating the needle, and frustrating the procedure.

This problem has caused some physicians to resort to inserting the injection catheter in a backward fashion (i.e., hub end first starting from the distal end of the endoscope) prior to placement of the endoscope within the patient. This is an undesirable solution to the problem for many reasons. First, the operative channel of the endoscope is rendered unavailable for use for other purposes (such as biopsy) for the duration of the procedure. Second, the syringe attachment means must be removed before the proximal end of the catheter is fitted through the endoscope, and then subsequently reattached. Third, extreme care must be exercised in maintaining proper placement of the catheter while the endoscope is inserted into the patient; misalignment would result in the needle catching on either the endoscope or patient during insertion.

It is clear from the foregoing that an improved device is needed; one which can be easily passed through the proximal end of the endoscope, at will, after the endoscope has been properly located within the patient.

It is therefore an object of the present invention to provide an improved catheter assembly which permits a catheter needle to be safely passed through a hollow passage without snagging on the passage walls.

It is a further object of the present invention to provide a method and apparatus whereby an inner, flexible catheter tube can take advantage of the increased rigidity inherent in two concentric tubes during placement of the catheter needle and thereafter take advantage of the flexibility of the catheter tube to prevent inadvertent rupture at the injection site.

It is another object of the present invention to provide a method of translating a flexible catheter and needle through an endoscope, blood vessel, or other passage without snagging the needle on the passage walls using a protective sheath but wherein the distal end of the catheter can project from the distal end of the sheath to take advantage of the flexibility of the catheter to preclude inadvertent rupture at the injection site.

It is still another object of the present invention to provide an improved mechanism for mutually locking two telescopically related tubes in place while simultaneously sealing one tube from the other.

It is still a further object of the present invention to provide an improved positional locking mechanism for two telescopically related tubes without interrupting flow through the inner tube.

In accordance with these and other objects of the present invention a flexible inner catheter tube is longer than and telescopically slidable within a more rigid, outer catheter sheath tube. An injection needle, secured to and projecting from the distal end of the inner catheter tube, is protected during traversal of an endoscope or blood vessel by retracting it into the distal end of the outer sheath tube. Upon reaching the injection site the needle may be projected a small distance from the distal sheath end; hence, the greater rigidity of the larger diameter outer sheath tube may be used to minimize bending of the inner catheter tube during traversal of the endoscopic passage and during insertion of the needle into the tissue at the injection site. The outer sheath may be retracted after insertion of the needle at the injection site so that the flexible catheter tube minimizes rupture at the injection site by bending at the exposed portion of the flexible catheter.

A locking mechanism comprises an elastomeric washer slidably engaged about the inner catheter tube at a location beyond the proximal end of the sheath. By axially compressing and radially constraining the washer, the axial compression produces radial compression of the washer about the inner catheter tube to preclude its axial displacement relative to the outer sheath at any location along its length. In the preferred embodiment, the washer is housed in a cup-like member which receives a stem of a housing member in threaded engagement. The stem axially compresses the washer at the cup end wall while the cylindrical cup wall radially compresses the washer. The proximal end of the outer catheter sheath passes slidably through the housing member and stem and includes a flanged proximal end which engages the end of the stem to limit translation of the sheath in one direction relative to the housing member. When the stem compresses the washer in the cup-like member, the washer bears axially against the stem end serving to both positionally lock the sheath relative to the washer and to seal the annular opening surrounding the catheter tube at the proximal end of the sheath.

The foregoing objects and features of the present invention will become more fully apparent from the following description and the appended claims taken in conjunction with the accompanying drawings.

Figure 1 is a side view in partial section of a catheter assembly of the preferred embodiment of the present invention;

Figure 2 is a detailed view in partial section of the positional locking mechanism employed in the assembly of Figure 1 showing the mechanism disengaged;

Figure 3 is a detailed view in partial section of the locking mechanism of Figure 2 showing the mechanism in locked position;

Figure 4 is a schematic sectional view showing the distal end of the catheter assembly of Figure 1 traversing an operative channel of an endoscope or other tubular passage; and

Figure 5 is a sectional view showing the distal end of the catheter assembly of Figure 1 projecting from the distal end of the tubular passage.

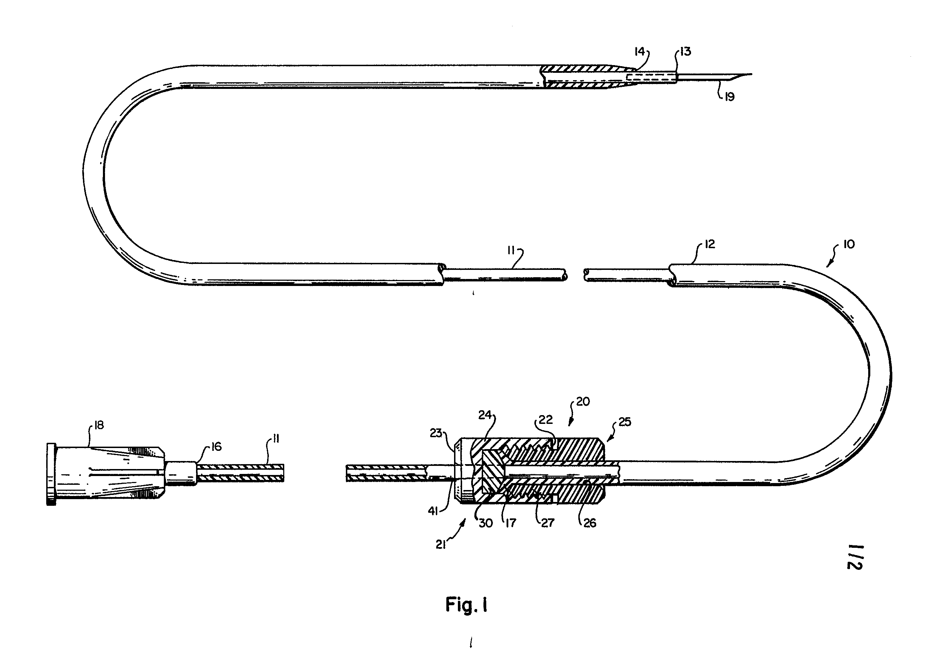

Referring to Figure 1 of the accompanying drawings in greater detail, a catheter assembly 10 includes an inner catheter tube 11 disposed for telescopic axial displacement in an outer catheter sheath tube 12. The inner tube 11 is considerably longer than the sheath tube 12 so as to permit the distal end 13 of the inner catheter tube 11 to project beyond the distal end 14 of the sheath tube. The proximal end 16 of the inner tube 11 also is long enough to extend beyond the proximal end 17 of the sheath tube 12. By way of example only, the sheath tube 12 may have a length of 150 cm whereas the inner catheter tube 11 would have a length of 225 cm; these dimensions have been employed successfully in a working embodiment.

The inner catheter tube 11 is telescopically translated axially within sheath tube 12 by grasping inner tube 11 between its proximal end 16 and the proximal end 17 of sheath tube 12 and pushing or pulling the inner tube with an axial force necessary to effect displacement either forward or backward, as desired. In this regard, the diametric clearance between the catheter tube 11 and sheath tube 12, as well as the materials of which they are made, are quite important. Excessive diametric clearance permits the inner catheter tube 11 to bend too much when it is pushed through the outer sheath tube 12 with the result that the inner catheter tube 11 tends to bunch up and kink, which impedes flow therethrough. This may also occur from axially applied pushing forces during tissue puncture with the catheter needle. On the other hand, the diametric clearance must be sufficient to permit the catheter tube to slide freely within the sheath tube. In the aforesaid working embodiment, by way of example only, the sheath tube 12 had an outside diameter of 0.085 inches and an inside diameter of 0.050 inches; the inner catheter tube 11 had an outside diameter of 0.044 inches and an inside diameter of 0.024 inches. The resulting 0.006 inches of diametric clearance (i.e., a 0.003 annular width clearance) proved successful in permitting smooth telescopic translation without kinking.

The materials used for the inner catheter tube 11 and sheath tube 12 affect the rigidity of the two tubes and the sliding frictional forces acting between them. Since the inner catheter tube 11 is relatively small in transverse section relative to its length, it tends to kink in the portion between application of the axial pushing force and the proximal end 17 of sheath tube 12. Thus, although inner tube 11 must be flexible to permit it to be extended through curved and tortuous passages, it must have sufficient rigidity to prevent such kinking. On the other hand, this kinking tendency is greater if the friction between the tubes is too resistive to axial pushing forces applied to the catheter tube. Therefore, apart from diametric clearance and catheter type flexibility considerations, the coefficients of friction for the tube materials are important. For the aforesaid working embodiment it has been found that polyethylene is a good choice of material for sheath tube 12; polytetrafluoroethylene (Teflon) was used for the inner catheter tube 11. One alternative material for the inner tube 11 would be stiff vinyl.

The proximal end 16 of inner catheter tube 11 terminates in a female fitting (such as a Luer fitting) adapted to receive a connection from a supply of fluid under pressure to be delivered by the inner catheter tube. A hollow injection needle 19 is press fit or otherwise bonded into the distal end 13 of the inner catheter tube 11 so as to project coaxially therefrom with a portion of the needle residing within the catheter tube. In the aforesaid preferred embodiment a 22 gauge stainless steel needle was employed with approximately 0.25 inches exposed from distal catheter tube end 13.

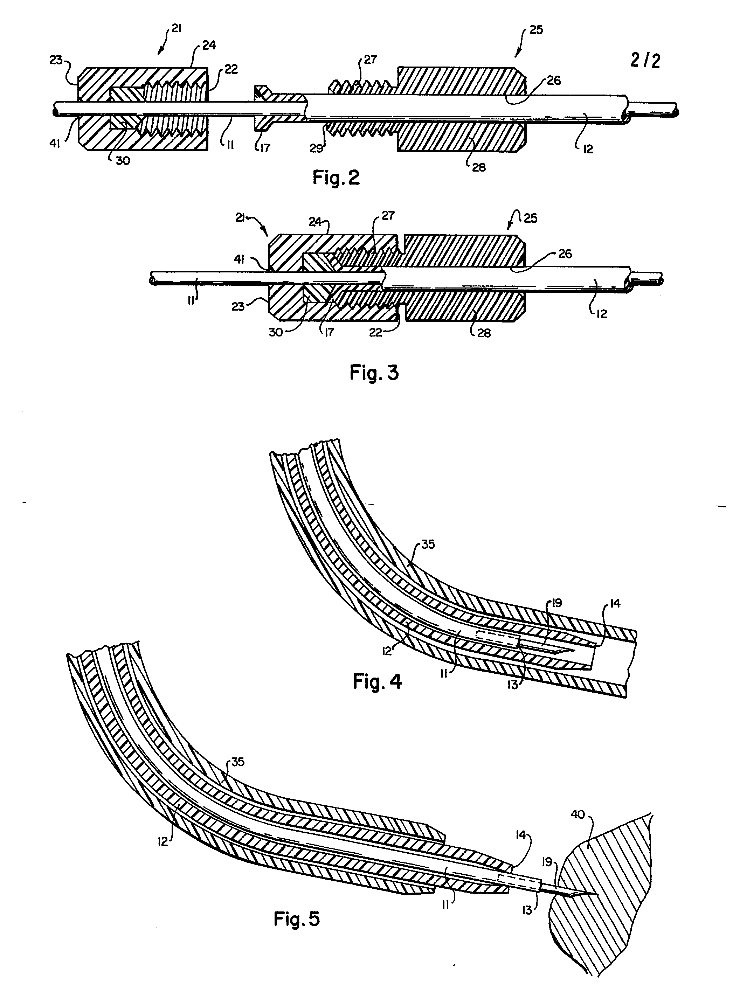

It is important that the catheter assembly 10 include a locking mechanism whereby the relative axial positions of the tubes 11, 12 can be locked or fixed selectively over a continuous range of such positions. A positional locking collar generally designated at 20 is provided for effecting such locking. Locking collar 20 is illustrated in greater detail in Figures 2 and 3 to which specific reference is now made along with Figure 1.

Cap 21 is a cup-like member having an open end 22, an opposite end wall 23 and a generally cylindrical side wall 24. End wall 23 has an aperture 41 centrally defined therein which is adapted to slidably engage the inner catheter tube 11 between its proximal end 16 and the proximal end 17 of the outer sheath tube 12. The open end 22 of cap 21 faces the proximal end 17 of sheath tube 12. Cylindrical wall 24 is internally threaded to engage an externally threaded stem portion 27 of a housing member 25.

Housing member 25 includes a longitudinal throughbore 26 in which sheath tube 12 is slidably engaged. Stem portion 27, which has a smaller outside diameter than the main housing body 28 from which it extends, has its end surface 29 contoured to match the contour of the generally radially-extending flange at the proximal end 17 of sheath tube 12. End surface 29 thus serves as a stop past which sheath tube 12 cannot be pulled through housing member 25 in one direction (to the right, as viewed in Figures 1, 2 and 3).

An elastomeric washer 30, made of rubber or similar material, is slidably mounted on the inner catheter tube 11 at a location between the end wall 23 of cap member 21 and the flanged proximal end 17 of sheath tube 12. In the preferred embodiment, washer 30 is snuggly retained against the interior surface of end wall 23 which, along with adjacent portions of sidewall 24 is contoured to receive the matching washer contour in a friction fit. Washer 30 is annularly shaped and preferably has an axial length which gradually decreases toward its radial extremity. The central aperture in the washer has a diameter slightly larger than the outside diameter of catheter tube 11 so that, when washer 30 is free of radial compression, tube 11 can slide freely through the washer aperture.

When the cap member 21 and housing member 25 are disengaged (as illustrated in Figure 2), or when these members are partially engaged without exerting axial pressure on washer 30, catheter tube 11 slides freely relative to washer 30, cap 21, housing 25 and sheath tube 12. When cap 21 and housing 25 are fully engaged, with stem 27 exerting axially compressive forces on washer 30 in cup-like member 21, the peripherally constrained washer tends to expand slightly in a radially inward direction. This expansion is not enough to significantly restrict fluid flow through the inner catheter tube 11 but is sufficient to frictionally engage the inner tube 11 to prevent it from sliding through the washer 30. This condition is illustrated in Figures 1 and 3.

Since the flanged proximal end 17 of the sheath is situated between the end surface 29 of stem 27 and the leading end of washer 30, the flanged end 17 is preferably contoured, as shown, to match the portions of the leading end of washer 30. This enhances the peripheral constraints on the washer 30 when it is axially compressed so that radially inward compressive forces can be more efficiently exerted by the washer 30 on the inner catheter tube 11. Additionally, the flanged end 17 is designed to provide a smooth, anti-frictional surface against which the stem 27 may easily turn.

Apart from locking the inner catheter tube 11 in place relative to washer 30, the axially compressive forces applied by virtue of full engagement between cap 21 and housing stem 27 act to positionally lock the flanged proximal end 17 of sheath tube 12 against end surface 29 of stem 27. At the same time, washer 30 seals the proximal end 17 of sheath tube 12. Therefore, the simple action of threadingly engaging cap 21 and housing stem 27 has the following effects: (a) the washer 30 is compressed by stem 27 so that the inner catheter tube 11 is positionally fixed; (b) sheath tube 12 is sealed at its proximal end 17 by the leading end of washer 30; and (c) the outer sheath tube 12 is positionally fixed relative to the inner

catheter tube 11. It should be noted that this multiple effect, achieved with a single threaded engagement, requires only three parts, namely: cap member 21, housing member 25 and washer 30.

The locking collar 20 described above has a number of other advantages. The collar effectively terminates the proximal end of the sheath tube 12 which, in the preferred embodiment, is made of polyethylene. This eliminates the need for conventional bonding procedures between the collar and sheath tube. Further, locking collar 20 provides for positional locking over substantially the entire length of inner tube 11. In addition, locking is achieved without kinking or damaging the inner catheter tube and without restricting flow therethrough. As noted above, locking collar 20 also provides a fluid tight seal between tubes 11 and 12.

Figure 4 illustrates the distal end of the catheter assembly of Figure 1 traversing a tubular passage 35 such as an operative channel in an endoscope. The catheter tube 11 is shown retracted into sheath 12 and locked in position so that catheter needle 19 and distal end 13 of the catheter tube are disposed rearward of the distal end 14 of the sheath. The needle is therefore protected by sheath 12 from snagging or puncturing the endoscope

0086338

during translation of the sheath or catheter. Upon reaching the location of intended tissue puncture, sheath tube 12 is extended somewhat beyond the distal end of the endoscope, as seen in Figure 5. For this purpose the sheath should obviously be made somewhat longer than the endoscope. The inner catheter tube 11 is then unlocked by unscrewing cap 21 and inner tube 11 is pushed while holding the sheath tube 12 until the sharpened tip of needle 19 extends beyond distal end 14 of the sheath. The catheter tube 11 is then locked again by screwing the stem 27 into cap 21, and the assembly is advanced until needle 19 punctures tissue 40.

It is advantageous to use the greater rigidity of the sheath tube 12 in applying the puncture force to prevent the smaller, more flexible inner catheter tube 11 from buckling if it alone were used to transmit the puncture force. After the puncture is made catheter tube 11 is again unlocked and sheath 12 may be retracted by pulling on locking collar 20 while holding catheter tube 11 steady, to expose a desired length of catheter tube 11 beyond the distal end 14 of the sheath. The desired exposed length of catheter tube 11 thus provides a degree of flexibility required at the puncture location to prevent rupture from inadvertent lateral movement of the needle.

Although the preferred embodiment of the present invention is described for transendoscopic applications, such as transendoscopic sclerosing of esophageal varices while under balloon tamponade, it will be appreciated that additional applications exist in any situation where it is desired to inject a fluid substance into a biological membrane or tissue at a location distal from the fluid pressure source and where the preferred method of transport of the needle and its attendant catheter tube is through the lumen of a hollow, tubular structure such as a blood vessel or the operative channel of an endoscope.

The invention may be embodied in other specific forms without departing from its spirit or essential characteristics. The described embodiment is to be considered in all respects only as illustrative and not restrictive and the scope of the invention is, therefore, indicated by the appended claims rather than by the foregoing description. All changes which come within the meaning and range of equivalency of the claims are to be embraced within their scope.

0086338

C L A I M S

1. A catheter assembly, characterized in that it comprises a sheath tube (12) having an open proximal end (17) and an open distal end (14), and an inner catheter tube (11) having a proximal end (16) and a distal end (13), wherein

said inner catheter tube (11) has a length greater that the length of said sheath tube (12) and an outside diameter smaller that the inside diameter of said sheath tube (12), said inner catheter tube (11) being disposed within said sheath tube (12) for telescopic movement therein to project the distal end (13) of the catheter tube (11) beyond the distal end (14) of the sheath tube (12) in response to application of axially-directed forces to said inner catheter tube (11),and wherein actuable locking means (20) are provi- ded for selectively preventing axial movement of said inner catheter tube (11) relative to said sheath tube (12) over a continuous range of axial positions of the inner catheter tube (11) relative to the sheath tube (12).

2. A catheter assembly according to claim 1, characterized in that it further comprises a hollow injection needle (19) secured to and projecting outwardly from said distal end (13) of said inner catheter tube (11) and wherein the distal end (13) of the inner catheter tube (11) is telescopically retractable into the distal end (14) of the sheath tube (12) a sufficient distance to permit the entire needle (19) to be withdrawn into the sheath tube (12).

3. A catheter assembly according to claim 1, characterized in that said sheath tube (12) is made of polyethylene and said inner catheter tube (11) is made of Teflon.

4. A catheter assembly according to claim 1, characterized in that said sheath tube (12) is made of polyethylene and said inner catheter tube (11) is made of vinyl.

5. A catheter assembly according to claim 1, characterized in that said locking means comprise:

an annular elastomeric washer (30) disposed in axially slidable engagement about said inner catheter tube (11) at a location beyond the proximal end (17) of said sheath tube (12); and

means (21, 25) for compressing said elastomeric member (30) to preclude relative axial movement between said catheter tube (11) and said elastomeric washer (30) while permitting free flow of fluid through said catheter tube (11).

6. A catheter assembly according to claim 5, characterized in that said further means comprise:

a housing member (25) disposed about said sheath tube (12), said housing member (25) having a throughbore (26) to permit axial sliding of said sheath tube (12) therein, said housing member (25) further including a stem portion (27); and

a cap (21) disposed about said elastomeric washer (30) and said inner catheter tube (11) to permit axially slidable movement of said inner catheter tube (11) through said washer (30) and said cap (21), said cap (21) having an open end for engaging said stem portion (27) in a locked position wherein said stem portion (27) axially compresses said elastomeric washer (30) so that it fixes the position of said inner catheter tube (11).

7. A catheter assembly according to claim 6, characterized in that said cap (21) is a hollow, generally cup-like member having a generally cylindrical wall (24) disposed concentrically about said inner catheter tube (11), and an end wall (23) opposite said open end with an aperture (41) through which said inner catheter tube (11) slidably extends, and in that said cylindrical and end walls (24,23) both axially and radially compress said elastomeric washer (30) when it is axially forced against said end wall (23) by said stem portion (27).

8. A catheter assembly according to claims 6 and 7, characterized in that said sheath tube (12) is flanged at its proximal end (17) so that it engages the stem portion (27) of said housing (25), and in that said flanged end (17) of said sheath tube (12) is forced against said elastomeric washer (30) to lock said sheath tube (12) against axial movement relative to said inner catheter tube (11) when said stem portion (27) is fully inserted into said open end of said cup-like member (21).

9. A catheter assembly according to claim 8, characterized in that said open end of the cup-like member (21) is threaded at its interior surface and in that said stem portion (27) is correspondingly threaded to mate with the said threaded interior surface of said cup-like member (21).

10. A catheter assembly according to claim 5, characterized in that it further comprises an endoscope (35) and in that said sheath tube (12) is longer than said endoscope (35), said sheath tube (12) being slidable through said endoscope (35).

11. A catheter assembly according to claim 5, characterized in that it further comprises a fitting (18) secured to the proximal end (16) of said inner catheter tube (11) for receiving a mating member connected to a source of liquid under pressure.

12. A catheter assembly, characterized in that it comprises a sheath tube (12) having an open proximal end (17) and an open distal end (14);

a flexible inner catheter tube (11) having a proximal end (16), a distal end (13), a length greater than the length of said sheath tube (12) and an outside diameter smaller than the inside diameter of said sheath tube (12), said inner catheter tube (11) being disposed within said sheath tube (12) for telescopic movement therein to project the distal end (13) of the catheter tube (11) beyond the distal end (14) of the sheath tube (12) in response to application of axially-directed forces forces to said inner catheter tube (11);

a housing member (25) disposed about said sheath tube (12), said housing member (25) having a throughbore (26) to permit axial sliding of said sheath tube (12) therethrough, said housing member (25) further including a stem portion (27);

an annular , elastomeric member (30) disposed in axially slidable engagement about said inner catheter tube (11) at a location beyond the proximal end (17) of said sheath tube (12); and

a cup member (21) disposed about said elastomeric member (30), one end of said cap member (21) having an aperture to permit axially sliding movement of said inner catheter tube (11) through said cap member (21) and said elastomeric member (30), said cap member (21) having an open end for engaging said stem portion (27) in a locked position wherein said stem portion (27) axially compresses said elastomeric member (30) so that it will grip the inner catheter tube (11) and fix its position relative to the outer sheath tube (12).

13. A locking mechanism for selectively preventing a first tube (11) from moving telescopically within a second tube (12), characterized in that it comprises:

an elastomeric member (30) disposed in axially slidable engagement about a portion of said first tube (11);

a housing member (25) disposed about said second tube (12), said housing (25) having a throughbore (26) to permit axial translation of said second tube (12) within said housing member (25);

a cap member (21) disposed about said elastomeric member (30) and said portion of said first tube (11), said cap member (21) having an aperture in one end thereof to permit axial translation of said first tube (11) therethrough; and

means for engaging said cap member (21) and housing member (25) in a locked position wherein said housing member (25) compresses said elastomeric member (30) so that it fixes the position of said first tube (11) relative to said second tube (12).

14. A locking mechanism according to claim 13, characterized in that said cap member (21) has a generally cylindrical wall (24) disposed concentrically about said first tube (11), an open end and an opposite end wall (23) with said aperture through which said first tube (11) slidably extends, and in that said end wall (23) is configured to both axially and radially compress said elastomeric member (30) when that member is axially forced against said end wall (23).

15. A locking mechanism according to claims 13 and 14, characterized in that said housing member (25) includes a stem portion (27) configured to permit it to be inserted axially into said cap member (21) through said open end, and in that said second tube (12) is flanged at its distal end (17) so that it engages the said stem portion (27), the flanged end engaging said elastomeric member (30) to lock said second tube (12) against axial movement relative to said housing member (25) when said stem portion (27) is fully inserted into the open end of said cap member (21).

16. A locking mechanism according to claim 15, characterized in that said cap member (21) includes a threaded interior surface of said generally cylindrical wall (24) and in that said stem portion (27) includes a threaded periphery for engaging the threaded interior surface.

17. A locking mechanism according to claim 13, characterized in that said first tube comprises an inner catheter tube (11) and in that said second tube comprises a sheath tube (12) which together form a catheter assembly, the assembly further comprising a hollow injection needle (19) secured to and projecting outwardly from the distal end (13) of said inner catheter tube (11) and in that the distal end (13) of the inner catheter tube (11) is telescopically retractable into the distal end (14) of the sheath tube (12) a sufficient distance to permit the entire needle (19) to be withdrawn into the sheath tube (12).

18. A method of inserting a fluid-delivering catheter according to anyone of claims 1 to 12 to an internal body part (40) of a living organism by means of passing a flexible catheter tube (11) having a hollow needle (19) projecting from its distal end (13) through a tubular passage (35) such as an endoscope or a blood vessel, said method being characterized in that it comprises the steps of:

traversing said tubular passage (35) with a sheath tube (12) concentrically disposed about said catheter tube (11) and with the distal end (13) of said catheter tube (11) and said needle (19) retracted and locked in position within the distal end (14) of the sheath tube (12);

unlocking said catheter tube (11) to permit axial movement of said catheter tube (11) relative to said sheath tube (12), and

telescopically translating said catheter tube (11) within said sheath tube (12) by applying axial force to the catheter tube (11).

19. A method according to claim 18, characterized in that it further comprises the step of:

positionally locking said catheter tube (11) relative to said sheath tube (12) to preclude further telescopic translation between said tubes (11, 12).

20. A method according to claim 19, characterized in that the step of positionally locking includes the steps of:

axially compressing an elastomeric member (30) disposed in concentrically slidable engagement about said catheter tube (11); and

radially compressing said elastomeric member (30) to tightly grip said catheter tube (11) and preclude axial translation of the catheter tube (11) relative to said sheath tube (12) while still permitting free fluid flow through said catheter tube (11).

21. A method according to claim 19, characterized in that it further comprises the steps of:

supporting the neelde (19) and distal end (13) of the catheter tube (11) with said sheath tube (12) during insertion of said needle (19) into said body part (40) to prevent bending the catheter tube (11) during insertion;

thereafter unlocking the catheter tube (11) to to permit retraction of the sheath tube (12); and

retracting the sheath tube (12) relative to the catheter tube (11) after insertion of the needle (19) into the body part (40).

Fig. I

1/2

0086338

Fig. 2

Fig. 3

Fig. 4

Fig. 5

**PARTIAL EUROPEAN SEARCH REPORT**
which under Rule 45 of the European Patent Convention
shall be considered, for the purposes of subsequent
proceedings, as the European search report

European Patent
Office

00 86338

Application number

EP 83100329.8

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| X | <u>GB - A - 1 601 780</u> (P.H. PEVSNER) | 1,5 | A 61 M 25/00 |
| A | * Fig. 2,4; page 2, lines 92-104; page 3, lines 48-63 * | 6,7,9, 12-17 | A 61 B 1/00 |
| | -- | | |
| X | <u>US - A - 4 235 232</u> (G.D. SPAVEN et al.) | 1 | |
| A | * Fig. 4-6; column 4, line 12 - column 5, line 4 * | 11,12 | |
| | -- | | |
| X | <u>US - A - 3 970 089</u> (D.D. SAICE) | 1 | |
| | * Fig.; column 3, lines 8-29 * | | **TECHNICAL FIELDS SEARCHED (Int. Cl.³)** |
| | -- | | |
| P,X | <u>DE - A1 - 3 214 905</u> (THE KENDALL CO.) | 1,5,11 | A 61 B 1/00 |
| A | * Fig. 3; page 11 - page 12, first paragraph * | 6,7,9, 12-17 | A 61 M 5/00 |
| | | | A 61 M 25/00 |
| | -- | | |
| A | <u>GB - A - 2 063 679</u> (ABBOTT LAB.) | 1,2,5- 7,11- 14,17 | |
| | * Fig. 4E; page 4, lines 89-106 * | | |
| | -- | | |

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 1-17
Claims searched incompletely: —
Claims not searched: 18-21
Reason for the limitation of the search:

In order to Art. 52(4)

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| VIENNA | 24-05-1983 | LUDWIG |

EPO Form 1505.1 06.78

European Patent Office

**PARTIAL EUROPEAN SEARCH REPORT**

Application number

0086338

EP 83100329.8

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| A | GB - A - 1 289 038 (LE VOY'S INC.)<br>* Fig. 1; page 2, lines 71-87 *<br>-- | 1,3,4 | |
| A | US - A - 4 254 762 (I. YOON)<br>* Totality; especially fig. 7 *<br>-- | 10 | |
| A | US - A - 4 222 380 (T. TERAYAMA)<br>* Totality *<br>---- | 10 | |

TECHNICAL FIELDS SEARCHED (Int. Cl.³)